(19) **Europäisches Patentamt**

**European Patent Office**

**Office européen des brevets**

(11) Publication number: **0 470 872 B1**

## EUROPEAN PATENT SPECIFICATION

(45) Date of publication of patent specification: **12.10.94**  (51) Int. Cl.<sup>5</sup>: **B01J 13/10**, B41M 5/165

(21) Application number: **91307418.3**

(22) Date of filing: **12.08.91**

The file contains technical information submitted after the application was filed and not included in this specification

(54) **Polysaccharide article and use thereof.**

(30) Priority: **10.08.90 US 567045**

(43) Date of publication of application:
**12.02.92 Bulletin 92/07**

(45) Publication of the grant of the patent:
**12.10.94 Bulletin 94/41**

(84) Designated Contracting States:
**AT BE CH DE DK ES FR GB GR IT LI LU NL SE**

(56) References cited:
**EP-A- 0 101 891**
**EP-A- 0 273 823**
**WO-A-88/08747**
**FR-A- 1 599 886**

(73) Proprietor: **Alko Ltd.**
**P.O. Box 350**
**SF-00101 Helsinki (FI)**

(72) Inventor: **Rha, Chokyun**
**285 Commonwealth Avenue**
**Boston, MA 02118 (US)**
Inventor: **Timonen, Maritta**
**Kantelettarentie 8E 57**
**SF-00420 Helsinki (FI)**

(74) Representative: **Collier, Jeremy Austin Grey et al**
**J.A.KEMP & CO.**
**14 South Square**
**Gray's Inn**
**London WC1R 5LX (GB)**

EP 0 470 872 B1

**Description**

Encapsulation technologies have numerous applications in a variety of industries when it is desireable to isolate a particular material until required. Encapsulation makes possible, for example, the ability to mask unpleasant tastes, to protect substances such as unsaturated fats and oils from oxidation, to control the release of encapsulated material, to convert a liquid material into a free flowing powder, to control flow properties, and to separate reactive materials until a particular reaction is desired. The encapsulated material may be released by mechanical, physical or chemical processes such as increasing the pressure or shear, increasing the temperature, dissolving the capsule wall, or letting the internal phase diffuse through the capsule wall.

Simple coacervation is the phenomenon of phasing a soluble polymer, such as gelatin, out of solution in order to encapsulate dispersed oil globules or solid particles within a uniform, continuous coating. This coacervation can be achieved by controlling the pH, temperature or polymer concentration. A typical procedure is to add a solution of salt to a solution of gelatin containing the dispersed oil globules, thereby reducing the solubility of the gelatin causing it to coacervate around the surfaces of suspended oil globules and eventually precipitate out of the solution. The microcapsules may then be separated and dried.

Complex coacervation is a controlled reaction between two oppositely charged polymers to form an insoluble polymeric complex that will precipitate from solution. The most common combinations are gelatin and one of one or more ionic polymers, such as gum arabic, pectin, and microbial polysaccharides (e.g, gellan gum). In food products, gelatin-gum arabic coacervates have been used to encapsulate flavors for inclusion in cake mixes, chewing gum, confectionary and other products.

Complex coacervation, however, requires complicated steps and a strict control of process parameters such as pH and temperature. Although gelatin is used for most of the complex coacervation procedures because it has good amphoteric properties as well as the ability to gel at temperatures above refrigeration, the structural network formed may not have sufficient structural integrity when high molecular weight ionic polysaccharides such as gum arabic, carboxymethyl cellulose, and synthetic ionic polymers are used. Conventional high molecular weight polymers used in encapsulation procedures have a number of other disadvantages. Such polymers give very high viscosity suspensions even at low concentrations and therefore limit the concentration of polymer that can be used in the encapsulation process. This makes the process difficult to carry out in practice. The capsules prepared using high molecular weight polymers are easily broken because the membrane has insufficient strength. In addition, the processing usually takes a long time, up to about 20 to 24 hours or even longer and requires a strict control of the processing conditions.

It is therefore important to provide a method of encapsulation which provides higher quality capsules and a higher yield of capsules. Such a method should be simple to operate and economical.

SUMMARY OF THE INVENTION

This invention relates to the use of polysaccharide derivatives of low molecular weight in encapsulation processes and the capsules thus produced.

The invention provides an insoluble polymeric capsule having at least two polymeric components, wherein one of said components is a hydrophilic colloid, and another of said components is a degradation product of a polysaccharide derivative, the said degradation product comprising a mixture of oligomers of which a majority have a degree of polymerization such that the oligomers conform to a rod-like configuration and have an average degree of polymerization in the range of about 3 to 100.

The invention also provides a method of making a polymeric capsule which comprises:

a) combining a hydrophilic colloid and a degradation product of a polysaccharide derivative, the said degradation product comprising a mixture of oligomers of which a majority have a degree of polymerization such that the oligomers conform to a rod-like configuration and have an average degree of polymerization in the range of about 3 to 100;

b) providing conditions sufficient for interaction between the hydrophilic colloid and the mixture of oligomers of step a) to form polymeric capsules; and

c) removing residual polymeric components from the capsules.

Methods of forming the capsules of the invention involve combining the stated polymeric components. One of the components is a mixture of oligomers derived from degradation of a polysaccharide derivative. The other component is a hydrophilic colloid which may be ionizable and oppositely charged and/or may be gellable. The mixture of oligomers has an average degree of polymerization in the range of about 3 to 100. The polysaccharide can be any one of a wide variety of polysaccharides although cellulose and starch are

2

preferred.

A substance to be encapsulated may be incorporated so that the capsules form around the substance. The substance can be introduced in any form capable of being encapsulated, e.g. solid, liquid or slurry. Residual polymer can then be removed. The membrane capsule may be strengthened by cross-linking a polymeric component of the capsule with a cross-linking agent, such as glutaraldehyde.

The method may comprise the step of adjusting the pH so that the hydrophilic colloid and the mixture of oligomers are similarly charged; introducing the selected substance to be encapsulated and forming an emulsion by beating or stirring; adjusting the pH of the mixture, if necessary, so that the ions of the said colloid and said oligomers have different electric charges and form droplets or capsules; cooling the emulsion to a temperature below the gelation point of the complex; washing the capsules to remove residual ionic polymer; if desired, hardening and strengthening the polymeric capsule by cross-linking the capsules with a cross-linking agent; separating the capsules from the remaining liquid, and, finally, drying them and comminuting them if aggregated. The adjustment or change in pH and lowering of temperature may not be necessary if the encapsulation is accomplished by a simple secondary interaction such as hydrogen bonding or Van der Waals forces.

The capsules of the invention are designed to contain any compatible substance. Such substances include pharmaceuticals, oils, synthetic oils, mineral oils, vegetable oils and animal oils. The encapsulated material can also be a fluid of an intrinsic color or ink that changes to a color when applied to sensitized record material.

The polysaccharide derivative may be degraded by enzymatic, chemical or physical or mechanical agents/mechanisms. In embodiments where an enzyme preparation is utilized to perform the degradation, the enzyme preparation is typically selected from the group of polysaccharide degrading enzymes. In the case of starch derivatives, enzymes such as amylases or pullulanases and mixtures thereof are suitable.

In embodiments where degradation of a polysaccharide derivative is to be effected by chemical or physical means, chemical hydrolysis, chemical oxidation and heat treatment are preferred mechanisms for achieving the desired polymeric mixtures according to the invention.

This invention further provides a pressure-sensitive paper to which is affixed oil, or ink containing capsules of the invention.

BRIEF DESCRIPTION OF THE FIGURES

Figure 1 schematically illustrates a method of forming capsules having a substance incorporated within the capsules.

Figure 2 is a photomicrograph of capsules formed using CMC hydrolyzates.

Figure 3 is a photomicrograph of capsules formed using high molecular weight CMC.

Figure 4 is a photomicrograph of capsules formed using CM Starch hydrolyzates.

Figure 5 is a photomicrograph of capsules formed using CM Starch.

DETAILED DESCRIPTION OF THE INVENTION

In broad outline, the method of the invention comprises combining at least two polymers under conditions sufficient to form capsules. One of the components may be an ionizable, hydrophilic material such as gelatin. The other component may be a charged polymer derived from at least one substituted polysaccharide, which polymer is derived from the degradation of the polysaccharide and has an average degree of polymerization in the range of about 3 to 100, and more preferably 5 to 50.

The term "polysaccharide" refers to a polymeric carbohydrate having a plurality of repeating units comprised of simple sugars. The term "polymeric" or "polymer" is meant to include both oligomeric and polymeric units and, preferably, those polysaccharides having more than four repeating monomeric simple sugar units.

The C-O-C linkage formed between two joined simple sugar units in a polysaccharide chain is called a glycosidic linkage, and continued condensation of monosaccharide units will result in polysaccharides. The most common polysaccharides are amylose and cellulose, both made up of glucose monomers. Amylose is a major constituent of starch and glycogen. Cellulose is the major structural component of plants. Other polysaccharides useful in this invention have a straight chain or branched polymer backbone including one or more sugar monomers. These polysaccharides include those having sugar monomers such as glucose, galactose, arabinose, mannose, fructose, rhamnose, and xylose.

Preferred polysaccharides useful in the article and methods of this invention are cellulose and starch. Nevertheless, examples of other such polysaccharides with branched or straight backbones are carrageenan, pullulan, pustulan, laminarin, scleroglucan, alginate, guar gum, gum arabic, inulin, pectin, whelan, rhamsan, gellan, xanthan, zooglan, methylan, chitin, cyclodextrin, and chitosan.

The term "derivative" is meant to define polysaccharides according to this invention that are substituted. Preferably, the polysaccharide derivative starting material has a degree of derivatization or substitution of between about 0.1 and about 3.0. "Degree of substitution" refers to the number of derivative groups (e.g. carboxymethyl, hydroxypropyl) per monomer unit in the polysaccharide backbone (branch or straight chain backbone). A degree of substitution of 0.2 means for example that there is about one derivative substituent for every five monomer units in the polysaccharide backbone. A degree of substitution of three would mean there are three derivative substituents per every monomer unit in a polysaccharide chain. The term "substituent" is meant to include any group attached to the polysaccharide backbone that imparts some chemical and/or physical property to the polysaccharide or provides some functional effect.

The polysaccharide derivative preferably includes one or more substituents selected from carboxymethyl, methyl, hydroxypropyl, methylethyl, hydroxyethyl, hydroxymethylethyl, hydroxypropylmethyl, sulfate, carboxylic acid, carboxylic acid ester, and pyruvate.

Carboxylic acid is found in carragenan, alginate and pectin. Pyruvic acid is found in pectin, xanthan gum, zooglan, and methylan.

Specifically, carboxymethyl starch can be degraded enzymatically to produce corresponding starch hydrolyzates. Other typical suitable starch derivatives include hydroxypropyl, methylethyl and hydroxyethyl starches. The substituents are typically bonded to a starch glucose monomer unit at the 2, 3 and 6 positions. Most typically a starch starting material comprises between about 1% to 85% amylose and about 15% to 99% amylopectin.

Cellulose derivatives are commercially available. Examples of cellulose derivatives include methylcellulose (MC, Methocel MC, 64630, Fluka Chemie AG, CH-9470 Buchs, Switzerland), hydroxypropylmethylcellulose (HPMC, H-9262, Sigma Chem. Co., St. Louis, MO) and carboxymethyl cellulose (CMC 7MFD, Blanose, Hercules Chem. Co., 92507 Rueil-Malmaison Ceder, France) all have a degree of substitution between 0.1 and 3. Hydroxypropyl celluloses are also commercially available and suitable for use.

As described more fully herein, such polysaccharide derivatives may be degraded to polymeric mixtures of average DP between about 3 and about 100 by enzymatic, chemical or physical or mechanical agents/means. The polymeric mixtures are generally referred to as a "hydrolyzate". The term "degraded" refers to the procedure whereby polysaccharide derivatives are broken down into smaller polymeric units.

Exemplary enzymes for use in degrading certain of the above described polysaccharide derivatives are pectinases, lyases, xanthanases, lysozymes, chitinases and laminarinases. Exemplary enzymes which are suitable for degrading cellulose derivatives are various cellulases. They can be produced from a multitude of different microorganisms such as strains of Trichoderma, Aspergillus, Penicillium, etc. A selected microorganism strain is grown by conventional means in medium containing food grade materials such that the cellulases are produced, the microorganism is separated from the medium, the medium is collected and typically concentrated and dried. These enzymes can be used as such or in mixtures and they can be modified in many different ways known to those skilled in the art.

A polysaccharide derivative may be hydrolyzed by treating a substituted starch derivative with a solution of acid. Typical acid treatment solutions might contain acids such as sulphuric acid, hydrochloric acid, phosphoric acid, or mixtures of the foregoing. The concentration of the acid in the treatment solution and the treatment time and temperature may vary depending on the degree of degradation of the polysaccharide derivative which is desired. In any event where an acid hydrolysis treatment is utilized, the acid concentration and the treatment time and temperature is selected to produce a mixture of polymers having an average DP of between 3-100.

A selected polysaccharide (e.g. starch or cellulose) derivative may be degraded by oxidation with such agents as chlorine, oxygen or hydrogen peroxide. Such oxidative treatments and reaction conditions are well known in the art. It may also be possible to use physical methods like heat or mechanical shear treatment or sonication when cleaving the chain backbone of polysaccharide derivatives.

Whatever conventional chemical (hydrolytic, oxidative or otherwise) or physical treatments are employed, the conditions and the degree of treatment are selected such that the mixture of oligomers resulting from the initial treatment has an average DP in the range of about 3 to 100, and contains less than about 25%, preferably less than 10% by weight of mono- and di-saccharides.

The mixture of oligomers preferably has a molecular weight distribution such that the polydispersity index of the mixture is less than 2.

Enzymes which may be used with respect to capsules prepared with degraded starch derivatives, are various amylolytic enzyme preparations. They can be produced from a multitude of different microorganisms such as strains of Bacillus, Klebsiella, Clostridium, Aspergillus, Rhizopus. Typical commercially available enzyme preparations suitable for use herein are amylolytic preparations (such as alpha and beta amylases), pullulanases, and cyclodextrin glycosyltransferase (CGTase).

The polymers described above are used in the method of the invention to form capsules. The term "capsule" is broadly meant to define roughly spherical and hollow objects having walls formed of polymeric material capable of surrounding substances such as liquids, solids, or slurries.

The substance may act as a seed or nuclei to stimulate formation of the capsules. The capsules formed by the process of the invention can vary widely in size. The capsule may range from less than about 10 $\mu$ to about 500 $\mu$ in diameter with a probable wall thickness of about 2 $\mu$ to about 10 $\mu$. Capsules can be as large as millimeter size.

The most preferred method comprises mixing together at least two polymeric components. One of these components is the substituted polysaccharide polymers described above having an average degree of polymerization in the range of about 3-100. At least one other of these components may be an ionizable hydrophilic material having an opposite electric charge to the substituted polysaccharide polymers. The term "ionizable hydrophilic material" is meant to include ionizable materials. This material can be gellable. Examples of the ionizable hydrophilic materials used to form the capsules of the invention can include gellable substances such as gelatin, albumin, casein, alginates, agar, pectins, and gum arabic.

In general, the capsules are caused to have deposited within them substances designed to be encapsulated. These encapsulated substances are centrally located in the capsule. The term "substance" can include any material or any that is capable of producing a desired effect in the particular environment or of performing a desired function. Capsules of the invention can preferably contain oil or oil-phase substances. The term "oil" or "oil-phase substances" is meant to include any water immiscible fluid such as natural occuring oils (olive oil, coconut oil, castor oil, fish oil, animal oils, vegetable and mineral oils) as well as synthetic oils such as methyl salicylate, and the like.

Capsules of the invention can also include bioactive substances.

The expression "bioactive substances" as used herein broadly includes any compound, or mixture thereof, that can be delivered from the capsule to produce a beneficial result. The bioactive substances can be soluble or it can have limited solubility in the capsule, such as an oil phase, powder, or other solid form. The term "bioactive substance" includes pesticides, herbicides, germicides, biocides, algicides, rodenticides, fungicides, insecticides, antioxidants, plant growth promotors, plant growth inhibitors, preservatives, disinfectants, sterilization agents, catalysts, chemical reactants, fermentation agents, foods, animal feeds, food or animal feed supplements, nutrients, cosmetics, drugs, vitamins, sex sterilants, fertility inhibitors, fertility promotors, air purifiers, microorganism attenuators, and other agents that benefit the environment of use.

The term "drug" includes any physiologically or pharmacologically active substances that produce a localized or systemic effect or effects in humans and other animals.

The term "animal" includes, but is not limited to, mammals and primates. Examples includes domestic household, sport or farm animals such as sheep, goats, cattle, horses, pigs, for administering to laboratory animals such as mice, rats and guinea pigs, and to fishes, to avians, to reptiles and zoo animals.

The term "physiologically" as used herein denotes the administration of drug to produce normal levels of functions. The term "pharmacologically" denotes the study of the actions of pharmaceutical on living systems, including therapeutics, as defined in Dorland's Illustrated Medical Dictionary, 1974. Published by W. B. Sanders Co., Philadelphia, Pa.

The bioactive substances that can be incorporated into the capsules of the invention include inorganic and organic compounds without limitation, including drugs that act on the peripheral nerves, adrenergic receptors, cholinergic receptors, nervous system, skeletal muscles, cardiovascular, smooth muscles, blood circulatory system, synaptic sites, neuroeffector junctional sites, endocrine and hormone systems, immunological system, reproductive system, skeletal system, alimentary and excretory systems, inhibitory and histamine systems, and those materials that act on the central nervous system such as hypnotics and sedatives.

Examples of beneficial pharmaceuticals are disclosed in Remington's Pharmaceutical Sciences, 16th Ed., 1980, published by Mack Publishing Co., Eaton, Pa; and in The Pharmacological Basis of Therapeutics, by Goodman and Gilman, 6th Ed., 1980, published by the MacMillan Company, London; and in The Merck Index, 10th Edition, 1983, published by Merck & Co., Rahway, N.J.

The dissolved pharmaceutical can be an ionizable salt. Acceptable salts include, but are not limited to, hydrochlorides, hydrobromide, sulfate, laurylate, palmitate, phosphate, nitrate, borate, acetate, maleate, tartrate, oleate, salicylate, salts of metals, and amines or organic cations, for example quaternary ammonium.

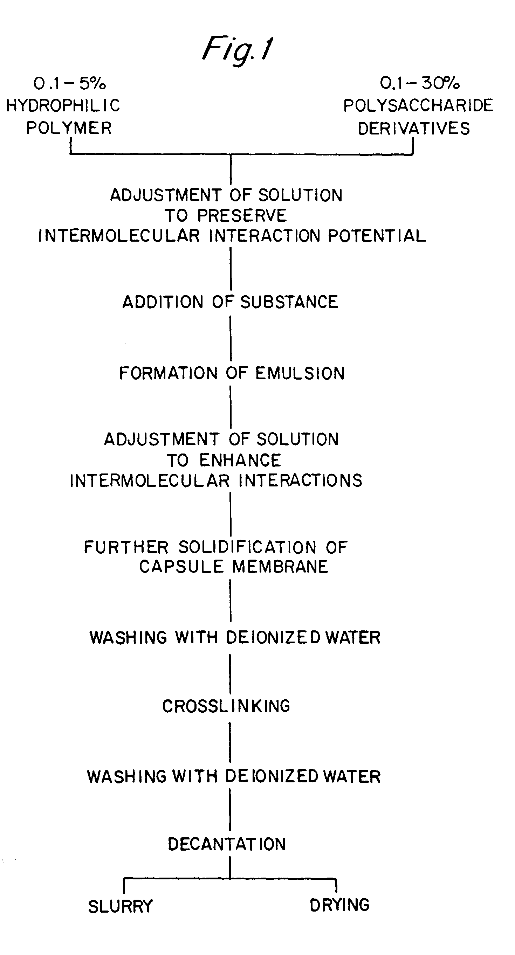

The process of the invention is shown in Figure 1. The method includes the steps of forming a mixture of at least two ionic polymers in water; at least one of the polymers may be an ionizable hydrophilic colloid and at least one other polymer is a mixture of charged polysaccharide derivatives, as described above; adjusting the pH to above the isoelectric point; introducing the selected substance to be encapsulated and forming an emulsion by beating or stirring; adjusting the pH of the mixture, if necessary, so that the ions of the two polymers have different electric charges and form coacervate droplets or capsules; cooling the emulsion to a temperature below the gelation point of the complex; washing the capsules to remove residual ionic polymer; if desired, hardening and strengthening the encapsulated material by cross-linking the capsules with a cross-linking agent; separating the capsules from the remaining liquid, and, finally, drying them and comminuting them, if aggregated.

In preferred embodiments, the charged polysaccharide derivatives described above are complexed with a gellable ionic polymer such as gum arabic or gelatin. Gelatin is particularly preferred for this procedure. Preferably, aqueous solutions of gelatin and charged polysaccharide polymer derivative hydrolyzates are mixed in the volume ratio of 1:1 at a pH above the isoelectric point. The pH can be adjusted with any compatible base, for example, sodium hydroxide or ammonium hydroxide, at a temperature above the gelation point of gelatin. Preferably, this temperature is between about 30°C and about 70°C depending on the molecular weight of the gelatin.

Concentrations of gelatin and charged polysaccharide derivative hydrolyzate solutions may vary from 0.1 to 5 percent (w/v) of gelatin and from about 0.1 to about 30 percent (w/v) solution of charged polysaccharide derivative hydrolyzate, depending on the average degree of polymerization.

Substances to be encapsulated is then added to the gelatin-polysaccharide solution in a volume ratio between about 1:2 to about 1:0.8. The solution is continually stirred or beaten during the incorporation of the substance to form a dispersion. When a dispersion is formed, the pH is reduced to about 3 or 4.5. Preferably, the pH is reduced to 4.0 using an acid such as hydrochloric acid. At this pH, gelatin acquires an overall positive charge which enables electrostatic interaction to occur between the gelatin and the polysaccharide derivative.

After adjusting the pH, the dispersion is cooled to about 10°C-30°C. Preferably, about 10 to about 15°C may be used.

The resulting capsules will gel at temperatures below the gelation point of the complex. Capsules formed by this procedure can then be washed with water to remove any residual gelatin. After this, the membrane of the capsule can be strengthened using various procedures.

Preferably, the gelatin component of the membrane is strengthened by cross-linking it with various cross-linking agents. Preferred cross-linking agents are dialdehyde, formaldehyde and glutaraldehyde. Solutions of cross-linking agents such as glutaraldehyde at 0.25 percent to 6 percent (w/v) final concentration are then added to the dispersion and the entire dispersion is vigorously stirred from 10 to about 20 minutes. The stirring, in combination with cross-linking agents promotes hardening of the capsules. Capsules can then be washed with water and dried.

An extremely wide variety of substances can be added to the mixture of charged polysaccharide derivative and gelatin. For example, food products such as fats, vitamins, minerals, and the like can be used. Synthetic oils, mineral oils, vegetable oils, and animal oils may be used such as, for example, methylsalicylate, petroleum oil, coconut oil, castor oil and sperm oil.

Other materials can be encapsulated by the method of this invention. Oils can be encapsulated that can be transferred to an underlying sheet by printing or marking pressure or heat that ruptures the capsules of the invention. "Printing or marking pressures" are meant to define art-recognised procedures for applying pressure. Examples include the use of pens, typewriters, computer-driven graphics, printers, and the like. The capsules are fixed to transfer film and pressure on the transfer film will cause marks on the underlying sheet. Examples of such oily printing fluids are chlorinated diphenyls as well as the oils mentioned above. Other dye materials can be incorporated into the oils used in transfer films such as Sudan III or Sudan IV.

Capsules produced by the method of the invention can be completely or partially coated on paper by commonly used procedures such as rolling, brushing or spraying. The capsules will adhere to the paper after drying.

In one embodiment, a sheet of paper is coated with the capsules of the invention. The capsules have within them an oily printing fluid combined with a colorless reactant that turns to a colored form on contact with a record material sensitized with a clay-like substance such as attapulgite or zeolite material. Methods of forming these pressure-sensitive papers are well-known in the art. See Green, U.S. Patent No. 2,712,507, incorporated herein by reference.

Thus the invention provides a paper product having coated on the surface thereof capsules containing a substance, for example a substance comprising an oil in combination with a colour reactant which turns to a coloured form on contact with appropriate record material, the capsules being rupturable by pressure applied to the coated paper.

The invention also provides a paper product having coated on the surface thereof the ruptured contents of the substance and capsules described above, the ruptured capsules optionally forming a continuous membrane on the surface of the paper product thereby forming a coated paper product.

Capsules of this invention can also be provided with oils for food preparation purposes. Capsules can be formed with, for example, cooking oil such as vegetable oil. Food to be cooked can be completely or partially coated with a myriad of these capsules. The heat of a broiling or baking oven can release the cooking oil directly from the interior of the capsule to the food that is to be cooked.

Furthermore, complete capsules or capsules ruptured by heat, pressure and the like may fuse together and completely or partially coat a material to form a continuous membrane on the surface of the material coated with the capsules. This membrane can improve mechanical, textural or functional properties of the product such as strength, taste, water retention and the like.

Thus the invention provides a method of food preparation comprising contacting capsules containing a substance, for example an oil or oil-phase, with a food; and releasing the substance located in the capsule thereby forming a food product containing the substance. Optionally the capsules are ruptured in a cooking step, are not removed and become part of the final product.

Capsules produced by the method of the invention can also be used to confine medicinal oils or bioactive substances, as defined above, to prevent them from being tasted upon being swallowed by a patient, to protect them from the deleterious influence of environmental conditions or from contamination.

The invention will now be more fully described by the following examples.

Example 1: Preparation of a Starch Precursor Hydrolyzate

Starch derivative hydrolyzates may be prepared from starch derivatives as defined above by an enzymatic hydrolysis utilizing an amylolytic preparation having α-amylase as the main active hydrolytic agent such that the only insignificant amounts of mono- and disaccharides are produced. The hydrolysis procedure is generally carried out by dissolving the starch derivative in water, adjusting the pH and the temperature to the value suitable for the enzyme activity, adding the enzyme to the solution and allowing the enzyme to react for a suitable time. After the enzyme reaction, the enzyme is inactivated by heating the solution up to about 100°C and the hydrolyzate product is concentrated and dried. The average degree of polymerization (DP) of the polymers obtained by such a hydrolysis is less than 500. The specific conditions suitable for and the specific time sufficient to secure the desirable degree of hydrolysis may be readily determined for each selected starch derivative and each selected enzyme preparation.

Similarly, where degradation is carried out using chemical or physical means, the average DP of the oligomers produced is less than 500.

60 g of carboxymethyl starch (CM starch) derived from potato starch (Primojel; Avebe, 9607 PT Foxhol, The netherlands) was mixed in 1200 ml of water. The temperature of the mixture was raised to 80°C and the suspension was mixed continuously. About 1.5 ml of amylase (Ban 120L, Novo, Industri A/S, Novo Alle, 2880 Bagsvaerd, Denmark) diluted 1/50 by volume was added to the suspension mixture. After hydrolysis of about 30 minutes the enzyme was inactivated by heating (100°C, 10 min.). The hydrolyzate was then freeze-dried.

The hydrolyzate contained negligible amounts of glucose, maltose and oligosaccharides, as the value of reducing sugars was 0.28%. The viscosity of a 5% by weight suspension of the hydrolyzate, measured using Haake-Rotovisco RV 12 viscometer with sensor systems NV; Karlsruhe, Federal Republic of Germany at 25°C was 57 mPa.s using the shear rate of 692 l/s. The viscosity of the unhydrolysed raw CM starch material was 106 mPa.s (25°C, 692 l/s). Since the viscosity of the hydrolysates is much lower, the hydrolysates can be used at at much higher concentrations than the original high molecular weight starch derivatives.

Example 2: Preparation of a Cellulose Precursor Hydrolyzate

Cellulose derivative hydrolyzates may be prepared from soluble cellulose derivatives as discussed above by an enzymatic hydrolysis utilizing a cellulase preparation having endo- 1, 4-beta-glucanase as the sole active hydrolytic agent such that only insignificant amounts of mono- and disaccharides which are absorbed in human intestine (e.g., glucose) or hydrolyzed by the intestinal bacterial flora (e.g., cellobiose), are produced. On the other hand the average degree of polymerization (DP) of the polymers formed by such a hydrolysis is less than about 500, and thus the viscosity of solutions of the hydrolyzate is reduced significantly compared to the viscosity of solutions of the unhydrolysed cellulose derivatives. The specific conditions suitable for and the specific time sufficient to secure the desired hydrolysis may be readily determined for each selected cellulose derivative and each selected enzyme preparation.

Similarly in other embodiments of the invention where degradation is carried out using chemical or physical means, the average DP of the polymers is less than 500 and the viscosity of the resulting mixture is significantly reduced.

The absolute amount of enzyme used in any particular example is hereafter reported in terms of the universal activity unit of nano-Katal (nkat). "nkat" refers to that amount of enzyme which produces one nanomole of reaction product in one second. Specifically the context of this application, nkat units refer to a hydrolyzate reaction product such as an polymer which is capable of reducing an agent such as dinitrosalicylic acid. The method of Bailey et. al., Enzyme Microb. Technol., Vol. 9, pp. 153-157 describes how such measurements of enzyme activity can be made using glucose as a standard.

Example 3: Preparation of Specific Cellulose Derivative Enzyme Hydrolyzates

a. Methylcellulose hydrolyzate

30 g of methylcellulose (MC, Methocel MC, 64630, Fluka Chemie AG, CH-9470 Buchs, Switzerland) was mixed in 3 l of water and the pH of the solution was adjusted to 5.5 with 15% phosphoric acid and the temperature was raised to 40°C. 0.3 ml of the enzyme preparation having a total endo-1, 4 beta-glucanase activity of 1680 nkat from which the beta-glucosidase activity was removed chromatographically (as described above) was added to the solution. After hydrolysis for 24 hours the enzyme was inactivated by heating (90°C, 15 min.). The hydrolyzate solution was subsequently cooled and freeze-dried.

The hydrolyzate product contained less than 0.5% by weight of glucose and cellobiose.

b. Hydroxypropylmethylcellulose hydrolyzate

20 g of hydroxypropylmethylcellulose (HPMC, H-9262, Sigma Chemical Company, St. Louis, MO, U.S.A.) was mixed in 1 l of water and the pH of the solution was adjusted to 5.5 with 15% phosphoric acid and the temperature was raised to 40° C. 0.24 ml of the enzyme preparation having a total endo-1, 4 beta-glucanase activity of 1340 nkat from which the beta-glucosidase activity was removed chromatographically (as described above) was added to the solution. After two hours another 20g of hydroxypropylmethylcellulose was added to the solution. After the hydrolysis of 22 hours the enzyme was inactivated by heating (90°C, 15 min.). Finally the hydrolyzate solution was cooled and freeze-dried.

The product contained less than 0.05% by weight of glucose and cellobiose.

c. Carboxymethylcellulose hydrolyzate

(i) Hydrolysis with Trichoderma reesei derived enzyme preparation

20 kg of carboxymethylcellulose (CMC 7MFD-type, a cellulose gum, also designated by the tradename Blanose and available from Hercules Chemical Company, 92507, Rueil-Malmaison Ceder, France; 7MFD designating a medium viscosity, food grade carboxymethylcellulose having 7 out of 10 glucose units substituted with carboxymethyl) was mixed in 320 l of water and the pH of the solution was adjusted to 5.5 with 15% phosphoric acid and the temperature was raised to 40°C. 0.27 l of the enzyme preparation having a total endo-1, 4 beta-glucanase activity of 1,780,000 nkat from which the beta-glucosidase activity was removed chromatographically (as described above) was added to the CMC solution. After one hour another 23 kg of CMC was added to the solution. After hydrolysis of 23 hours the enzyme was inactivated by heating (90°C, 15 min.). Finally, the hydrolysis solution was concentrated by conventional evaporating and spray-drying.

The product contained less than 2% by weight of glucose and cellobiose. When the same hydrolysis was carried out with the original cellulase enzyme preparation of Trichoderma reesei-fungus, the amount of produced glucose and cellobiose was above 5% by weight.

(ii) Hydrolysis with Aspergillus and Penicillium derived enzyme preparations

The enzyme preparations selected were commercially available Cellulase AP 3 (Amano Pharmaceutical Co., Ltd., Nagoya, Japan) produced using an Aspergillus strain and Cellulase CP (Sturge Enzymes, North Yorkshire, England) produced using a <u>Penicillium</u> strain. Carboxymethylcellulose hydrolyzates were prepared as described in Example c(i), except that 30g of CMC-7MFD was used in 1 l of water, and the amounts of enzymes added were 0.028 g of Cellulase AP 3 (having a total <u>endo</u>-1, 4 <u>beta</u>-glucanase activity of 1350 nkat) and 0.048 g of Cellulase CP (having a total <u>endo</u>-1, 4 <u>beta</u>-glucanase activity of 1350 nkat). The viscosities and molecular weight distributions of the hydrolyzates produced by either cellulase were similar to the hydrolyzate produced with enzymes derived from <u>Trichoderma reesei</u>.

The viscosities of the various cellulose derivatives and their hydrolyzates as described above were measured using a Haake-Rotovisco viscometer with sensor systems NV (Karlsruhe, Federal Republic of Germany) (Table 2). The viscosities were measured in water solutions at 25°C. Table 2 sets forth the concentrations (by weight) of a variety of solutions all having the same viscosity.

## TABLE 2

### Concentrations of cellulose derivatives and their respective hydrolyzates in solution all having a viscosity of 20 mPa.s (milli-Pascals-second) at 25°C.

| Cellulose Derivative | Concentration (by weight) |
|---|---|
| Methylcellulose | 2% |
| Methylcellulose hydrolyzate | 5% |
| Hydroxypropylmethylcellulose | 3% |
| Hydroxypropylmethylcellulose hydrolyzate | 10% |
| Carboxymethylcellulose | 2% |
| Carboxymethylcellulose hydrolyzate | 20% |

As the data in Table 2 indicates, the hydrolyzate of a cellulose derivative has a substantially lower viscosity than an equal amount by weight in aqueous solution of the cellulose derivative itself.

Example 4: <u>Carboxymethylcellulose Chemical Hydrolysis</u>

2 gms of carboxymethylcellulose (Blanose Cellulose Gum 7 LFD, Hercules Chemical Co., 92507, Rueil-Malmaison cedex, France) was hydrolyzed for about one hour in 100 ml of 1M sulphuric acid solution at about 100°C. After hydrolysis the solution was cooled to about room temperature, neutralized to about pH 6 with 25 ml of 25% (w/w) of NaOH solution and freeze-dried. This hydrolysis treatment produced a mixture of oligomers containing less than about 4% by weight of saccharides (cellobiose and glucose). The viscosity (and average DP) of this hydrolyzate is similar to the viscosities (and average DP) of the hydrolyzates produced by the enzymatic treatments described above utilizing enzymes derived from <u>Trichoderma reesei</u>.

Carboxymethyl cellulose (CMC) hydrolyzates can be prepared by enzymatic, chemical or physical methods as disclosed in U.S. Patent Applications Serial Nos. 07/309,387, 07/370,629 and 07/464,291. CMC hydrolyzates used in present invention have the average degree of polymerization in the range of 5 to 500, based on the viscosity average molecular weight. The viscosity average molecular weights of the CMC hydrolyzates were calculated using the Mark-Houwink equation:

$$[\eta] = KM_v^a$$

where $[\eta]$ is intrinsic viscosity, $M_v$ is the viscosity average molecular weight of the polymer and K and a are hydrodynamic constants caracteristic of the particular polymer-solvent system. The values of K and a for CMC, which were used in this study, were K = 0.043 in 0.2 M NaCl and a = 0.76 in 0.2 M NaCl as described in Brown and Henley, Studies on Cellulose Derivatives Part IV. The Configuration of the Polyelectrolyte in Sodium Chloride Solutions, Macromol. Chem., Vol. 79, 68-88 (1964). It is noted that a variety of methods for determining average molecular weights exist, and therefore the values of average molecular weights determined, as well as the average DP values calculated from them, depend upon the experimental method and the basis for calculation. CMC hydrolysates described in this invention have an intrinsic viscosity of between 50 ml. per gram to 3 ml. per gram, when determined in 0.2M sodium chloride. The CMC hydrolysates have the viscosity value in the range of from 5 to 100 mPa.s, when measured in 20% (by weight) solution at 25°C with shear rate $584s^{-1}$ using a Haake Viscotester, VI 500 with sensor system NV (Karlsruhe, Federal Republic of Germany).

The Mark-Houwink exponent, a, is indicative of the conformation of the polymer chain in solution. The conformation of the polymer chain in solution may be classified as an 1) impermeable dense sphere, 2) random coil, e.g. semi-permeable or free draining, and 3) rodlet or rod-like. Mark-Houwink exponents of 0.002 to about 0.5 correspond to dense spheres, exponents of about 0.5 to about 0.8 correspond to semi-permeable random coils, exponents of 0.8 to about 1.2 correspond to free draining random coils and exponents of about 1.2 to about 2 correspond to rodlets or rod-like oligomers or polymers.

In an embodiment of this invention, the degradation product of the polysaccharide derivative comprises a mixture of oligomers of the polysaccharide having a Mark-Houwink exponent of at least 1.5 at an NaCl concentration of about 0.005N to about 0.5N. This NaCL concentration range is typically used when measuring Mark-Houwink exponents. The salt content of foodstuffs may also typically fall into this range.

CMC raw material (Mw>15,000 Daltons) has Mark-Houwink exponents of 0.83-0.97, indicating a free draining random coil conformation. In the random coil conformation, polymer coils are confined by the intra-chain interactions; therefore less change is seen in the Mark-Houwink exponent within the same range of ionic strength. However, when the weight average molecular weight is less than 15,000 Daltons, the CMC chain is not sufficiently long to form a winding coil, the polymer chain is no longer subjected to the constraint of intra-chain interactions, and a chain of free strip or rod-like configuration may form. When the ionic strength is low, the electrostatic repulsion force becomes dominant due to the negative charge of the carboxymethyl groups, and the polymer assumes its most stiff rod-like conformation with the highest value of the Mark-Houwink exponents. When the ionic strength increases, the negative charge of carboxymethyl groups is shielded, the repulsion forces between the neighbouring groups are reduced, and the polymer chains relax, yielding a lower Mark-Houwink exponent.

The experimentally determined data show that the molecular weight and chain conformational characteristics of the most preferred cellulose derivative oligomeric mixtures used in the invention, i.e. mixtures comprising a significant or substantial portion of oligomers of rod-like conformation, are distinctly different from those of undegraded cellulose derivatives. As shown by the experimentally determined Mark-Houwink a values listed in Table 3 below for weight average molecular weights, $M_w$, of CMC at less than about 15,000 daltons (a = 1.58 to 2.07), the literature value of a = 0.74 for CMC is erroneous with respect to CMC having a $M_w$ of less than about 15,000 daltons. These experimentally determined data quantitatively indicate that relatively short chain CMC assumes a rod-like configuration as opposed to a free draining random coil conformation of the undegraded polymer.

## TABLE 3

## Mark-Houwink Equations for CMC (25°C)

| NaCl Concentration (N) | Weight Average Molecular Weight | |
|---|---|---|
| | >15,000 | <15,000 |
| 0.005 | $[\eta]=0.0069M_w^{0.97}$ | $[\eta]=0.02\times10^{-5}M_w^{2.07}$ |
| 0.010 | $[\eta]=0.0084M_w^{0.94}$ | $[\eta]=0.17\times10^{-5}M_w^{1.82}$ |
| 0.050 | $[\eta]=0.0090M_w^{0.91}$ | $[\eta]=0.83\times10^{-5}M_w^{1.63}$ |
| 0.100 | $[\eta]=0.0116M_w^{0.88}$ | $[\eta]=1.18\times10^{-5}M_w^{1.59}$ |
| 0.200 | $[\eta]=0.0182M_w^{0.83}$ | $[\eta]=2.00\times10^{-5}M_w^{1.55}$ |
| 0.500 | $[\eta]=0.0179M_w^{0.83}$ | $[\eta]=1.21\times10^{-5}M_w^{1.58}$ |

Furthermore, the most preferred oligomeric mixtures according to the invention have a relatively narrow range of molecular weights, i.e. relatively monodispersed, having a polydispersity index ($M_w/M_n$, weight average molecular weight divided by number average molecular weight) of less than about 2.0 and typically less than about 1.8. The weight average molecular weights and number average molecular weights of a variety of CMC hydrolysate samples of different degree of hydrolysis were measured and the polydispersity index of all such hydrolysates was calculated as ranging between about 1.1 and about 1.9. Therefore, the oligomers in a most preferred mixture of oligomers extend over a relatively narrow range of $M_w$ and, even as to mixtures having an average molecular weight at or near the upper limit of $M_w$ where the oligomers may begin to assume a random coil configuration, are comprised of a significant portion, preferably a majority, of oligomers having a rod-like configuration.

In the experimental determination of $M_w$ values, CMC solutions were prepared in 0.2N NaCl solution at pH of 7. The solutions were passed through an HPLC column, and the light intensity was detected by multiangle laser light scattering using a Wyatt Technology, multiangle laser light scattering instrument, model DAWN-F. The flow rate was 0.2 ml/min. The concentrations of the solutions were detected by refractometer, and the sensitivity of the refractometer was 64. The weight average molecular weights, $M_w$, were determined using appropriate computer software.

Example 5: Preparation of Capsules using Carboxymethyl Cellulose Hydrolyzates

1. One hundred ml of 4 percent (w/v) gelatin from porcine skin (bloom strength 175) was dissolved in distilled water and 100 ml of 1 percent (w/v) carboxylmethyl cellulose hydrolyzate having an intrinsic viscosity of 31.4 ml/g (average DP about 40) was prepared in distilled water at 60°C. The CMC hydrolyzate solution and gelatin solution were placed in a 1000 ml beaker, mixed, and the pH of the solution adjusted to 10. One hundred and fifty ml of soybean oil was added to the solution while stirring with a magnetic stirrer at 100 rpm. After forming a stable emulsion, the pH was adjusted to 4 to provide conditions for electrostatic interaction to take place between gelatin and CMC hydrolyzate.

The temperature of said solution was decreased to less than 15°C for the gelation of gelatin on the surface of the coacervated capsules. The capsules were washed with water to remove residual gelatin. Spherical capsules were formed with sizes ranging from 400 $\mu$ to 500$\mu$.

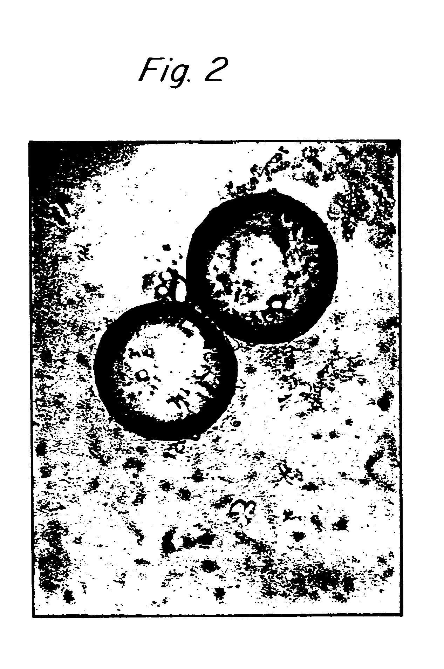

Capsules were then suspended in 1 percent glutaraldehyde solution and stirred for 15 minutes to strengthen the capsule walls. Capsules were washed with water and the water was decanted by using a strainer A capsule slurry was obtained and some portion of the capsule slurry was dried. The capsules have a thin membrane with wall thickess less than about 2$\mu$ (Figure 2).

2. Capsules were prepared with the same procedure as above but with high molecular weight CMC having an intrinsic viscosity of 280.4 ml/g (the average DP about 700) instead of CMC hydrolyzate.

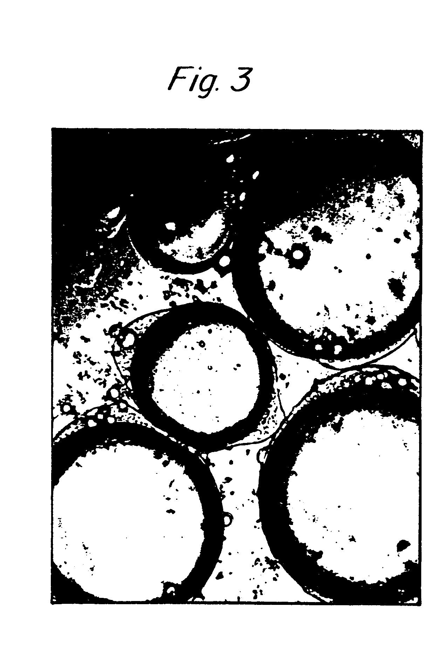

These capsules had a membrane with thickness more than 10μ and had irregularities on the membrane walls (Figure 3).

Capsules obtained in each example were subjected to following tests:

a) Sonication Test

To test for relative strength, the capsules were sonicated. Six ml of water and 0.1 ml of fully hydrated capsule slurry was mixed. After standing for 5 minutes, capsules were sonicated using Bransonic Ultrasonic cleaner Model 1200 (Danbury, CT) for 2 minutes. Ultrasonic treatment breaks the capsules and then releases the oil. The oil released is indicated by increased turbidity in the aqueous phase. The turbidity of the aqueous phase was measured by a spectrohotometer at 595nm. Higher turbidity indicates more broken capsules, therefore, more fragile and unstable capsule membranes.

b) Heating Test

This test provides relative values on the thermal stability and mechanical strength of the capsules. Known amount of capsules were spread on glass microscope slides and dried in a 50°C oven overnight. Then the sample was heated at 265°C for 20 minutes. The amount of oil loss was measured.

c) Suspended Volume Measurement

In order to measure the total volume of the capsules, prepared capsules were suspended quantitatively in 1000ml of water. The volume of the capsules suspended was determined in a graduated cylinder.

Results

Capsules prepared from carboxymethyl cellulose hydrolyzates (CMC hydrolyzates) had an absorbance of 0.86 at 595nm in the sonication test. Five percent of oil was lost as determined by the baking test, and the suspended volume was 217 ml. However, the capsules prepared using high molecular weight carboxymethyl cellulose had an absorbance of 1.30. Nine percent of oil was lost according to the baking test, and the suspended volume was 280 ml.

According to these tests, capsules prepared with low molecular weight hydrolyzates are more stable, have a stronger capsular membrane, occupy less volume and are more compact than capsules prepared with high molecular CMC. Therefore, capsules prepared with CMC hydrolyzate can contain more oil within a given volume with superior mechanical properties.

3. Carboxymethyl cellulose hydrolyzate capsules were prepared with 200 ml of oil.

4. High molecular weight carboxymethyl cellulose capsules were prepared with 200 ml of oil.

Test results indicate that capsules prepared with CMC hydrolyzate have thinner membranes and better mechanical properties than capsules prepared with high molecular weight CMC.

5. Carboxymethyl cellulose hydrolyzate capsules were prepared with 250 ml of oil.

6. High molecular weight carboxymethyl cellulose capsules were prepared with 250 ml of oil.

Capsules prepared with CMC hydrolyzate had a thin membrane and spherical shape with no leaking or rupturing of the capsules. However, the capsules prepared with high molecular weight CMC leak a considerable amount of oil with breaking of many capsules, probably due to lack of mechanical strength of the capsular membrane.

7. CMC hydrolyzate having an intrinsic viscosity of 18.6 ml/g, (the average DP about 20) were used to prepare the capsules.

8. CMC hydrolyzate capsules were prepared with gelatin having a bloom strength of 300.

9. CMC hydrolyzate capsules were prepared with two percent gelatin having a bloom strength of 175.

10. High molecular weight CMC capsules were prepared with two percent gelatin having a bloom strength of 175.

Test results indicate that the capsules prepared using carboxymethyl cellulose hydrolyzates are superior in mechanical property to those prepared with high molecular weight CM cellulose.

Example 6: Preparation of Capsules using Carboxymethyl Starch Hydrolyzates

The procedure of Example 5 was repeated with carboxymethyl starch hydrolyzate and with carboxymethyl (CM) starch of higher molecular weight (Primojel: Avebe, 9607 PT Foxhol, The Netherlands). Carboxymethyl starch hydrolyzate was prepared as described in Example 1.

1. Capsules were prepared using Carboxymethyl (CM) starch hydrolyzate.

2. Capsules were prepared using high molecular weight Carboxymethyl starch.

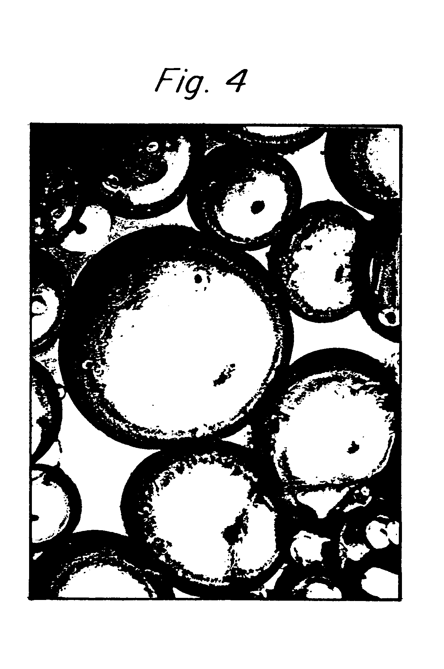

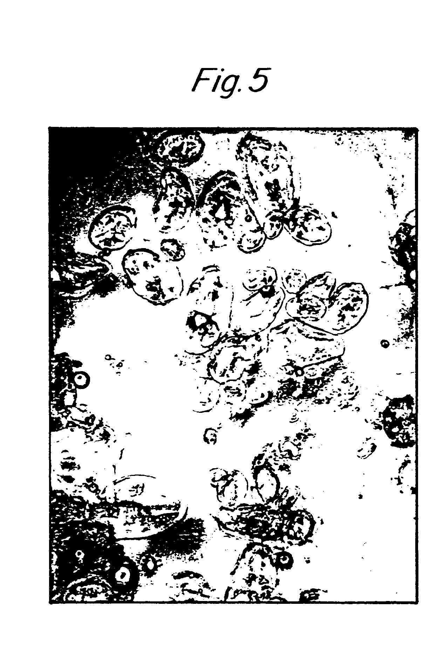

Capsules prepared using the carboxymethyl starch hydrolyzate had a fairly spherical shape of about 300$\mu$ diameter with thin membrane (Figure 4). However, the capsules prepared using carboxymethyl starch were mostly broken (Figure 5).

## Claims

**Claims for the following Contracting States : AT, BE, CH, DE, DK, FR, GB, GR, IT, LI, LU, NL, SE**

1. An insoluble polymeric capsule having at least two polymeric components, wherein one of said components is a hydrophilic colloid, and another of said components is a degradation product of a polysaccharide derivative, the said degradation product comprising a mixture of oligomers of which a majority have a degree of polymerization such that the oligomers conform to a rod-like configuration and have an average degree of polymerization in the range of about 3 to 100.

2. The capsule of claim 1, wherein the capsule has a wall thickness of less than about 10 microns.

3. The capsule of claim 1 or 2, wherein the polysaccharide derivative is a cellulose derivative, a starch derivative, carrageenan, pullulan, pustulan, alginate, laminarin, guar gum, gum arabic, inulin, pectin, whelan, rhamsan, gellan, xanthan, scleroglucan, zooglan, methylan, chitin, cyclodextrin or chitosan.

4. The capsule of claim 3, wherein the polysaccharide derivative includes one or more substituents selected from carboxymethyl, methyl, hydroxypropyl, methylethyl, hydroxyethyl, hydroxymethylethyl, hydroxypropylmethyl, sulfate, carboxylic acid, carboxylic acid ester, and pyruvate.

5. The capsule of any one of claims 1 to 4, wherein the mixture of oligomers has a molecular weight distribution such that the polydispersity index of the mixture is less than 2.

6. The capsules of any one of claims 1 to 5, wherein the mixture of oligomers contains less than 25% by weight of mono- and di-saccharide.

7. The capsule of any one of claims 1 to 6, further comprising a substance within the capsule.

8. The capsule of claim 7, wherein the substance is a bioactive substance and or an oil.

9. The capsule of claim 8, wherein the bioactive substance is a cosmetic, a food or a feed product.

10. The capsule of claim 8, wherein the oil is selected from animal oil, vegetable oil, mineral oil, and synthetic oil.

11. The capsule of any one of claims 1 to 10, wherein the hydrophilic colloid is capable of gelling, and is ionizable or non-ionizable.

12. The capsule of claim 11, wherein the gellable colloid is gelatin, albumin, gum arabic, an alginate, casein, agar-agar, or a pectin.

13. The capsule of claim 11 or 12 wherein the hydrophilic colloid has an opposite charge to the said mixture of oligomers.

14. The capsule of any one of claims 1 to 13, wherein the capsule membrane is 2 $\mu$ to 10 $\mu$ in thickness.

15. A method of making a polymeric capsule which comprises:
    a) combining a hydrophilic colloid and a degradation product of a polysaccharide derivative, the said degradation product comprising a mixture of oligomers of which a majority have a degree of polymerization such that the oligomers conform to a rod-like configuration and have an average degree of polymerization in the range of about 3 to 100;
    b) providing conditions sufficient for interaction between the hydrophilic colloid and the mixture of oligomers of step a) to form polymeric capsules; and
    c) removing residual polymeric components from the capsules.

EP 0 470 872 B1

**16.** The method of claim 15 wherein the hydrophilic colloid is as defined in claim 11, 12 or 13.

**17.** The method of claim 16 wherein the hydrophilic colloid has an opposite charge to the mixture of oligomers and the pH is adjusted in step b) so that ions of the said colloid and said oligomers have different electric charges and form coacervate droplets or capsules.

**18.** The method of claim 15, 16 or 17 wherein the said mixture of oligomers is as defined in any one of claims 3, 4, 5 or 6.

**19.** The method of any one of claims 15 to 18, further comprising combining the mixture of step a) with a substance to be encapsulated thereby forming capsules containing the substance.

**20.** The method of claim 19, wherein the substance is an defined in any one of claims 8 to 10.

**21.** The method of any one of claims 15 to 20, further comprising strengthening the insoluble polymeric complex by crosslinking the membrane with a crosslinking agent.

**22.** The method of claim 21, wherein the crosslinking agent is selected from glutaraldehyde, dialdehyde and formaldehyde.

**23.** A method of food preparation comprising contacting capsules of claim 7 with a food; and releasing the substance located in the capsule thereby forming a food product containing the substance.

**24.** A method as claimed in claim 23, wherein the substance is an oil or oil-phase.

**25.** A method as claimed in claim 23 or 24, wherein the capsules are ruptured in a cooking step, are not removed and become part of the final product.

**26.** A paper product having coated on a surface thereof the capsules of claim 7, the capsules being rupturable by pressure applied to the coated paper.

**27.** A paper product having coated on the surface thereof the ruptured contents of the substance and capsules of claim 7.

**28.** The paper product of claim 27, wherein the ruptured capsules form a continuous membrane on the surface of the paper product thereby forming a coated paper product.

**29.** The paper product of claim 26, wherein the substance within the capsule comprises an oil in combination with a colour reactant which turns to a coloured form on contact with appropriate record material.

**Claims for the following Contracting State : ES**

**1.** A method of making a polymeric capsule which comprises:
a) combining a hydrophilic colloid and a degradation product of a polysaccharide derivative, the said degradation product comprising a mixture of oligomers of which a majority have a degree of polymerization such that the oligomers conform to a rod-like configuration and have an average degree of polymerization in the range of about 3 to 100;
b) providing conditions sufficient for interaction between the hydrophilic colloid and the mixture of oligomers of step a) to form polymeric capsules; and
c) removing residual polymeric components from the capsules.

**2.** The method of claim 1, wherein the hydrophilic colloid is capable of gelling, and is ionizable or non-ionizable.

**3.** The method of claim 2, wherein the gellable colloid is gelatin, albumin, gum arabic, an alginate, casein, agar-agar, or a pectin.

14

4. The method of claim 2 or 3 wherein the hydrophilic colloid has an opposite charge to the said mixture of oligomers.

5. The method of claim 2, 3 or 4 wherein the hydrophilic colloid has an opposite charge to the mixture of oligomers and the pH is adjusted in step b) so that ions of the said colloid and said oligomers have different electric charges and form coacervate droplets or capsules.

6. The method of any one of claims 1 to 5, wherein the polysaccharide derivative is a cellulose derivative, a starch derivative, carrageenan, pullulan, pustulan, alginate, laminarin, guar gum, gum arabic, inulin, pectin, whelan, rhamsan, gellan, xanthan, scleroglucan, zooglan, methylan, chitin, cyclodextrin or chitosan.

7. The method of claim 6, wherein the polysaccharide derivative includes one or more substituents selected from carboxymethyl, methyl, hydroxypropyl, methylethyl, hydroxyethyl, hydroxymethylethyl, hydroxypropylmethyl, sulfate, carboxylic acid, carboxylic acid ester, and pyruvate.

8. The method of any one of claims 1 to 7, wherein the mixture of oligomers has a molecular weight distribution such that the polydispersity index of the mixture is less than 2.

9. The method of any one of claims 1 to 8, wherein the mixture of oligomers contains less than 25% by weight of mono- and di-saccharide.

10. The method of any one of claims 1 to 9, further comprising combining the mixture of step a) with a substance to be encapsulated thereby forming capsules containing the substance.

11. The method of claim 10, wherein the substance is a bioactive substance and/or an oil.

12. The method of claim 11, wherein the bioactive substance is a cosmetic, a food or a feed product.

13. The method of claim 11, wherein the oil is selected from animal oil, vegetable oil, mineral oil, and synthetic oil.

14. The method of any one of claims 1 to 13, further comprising strengthening the insoluble polymeric complex by crosslinking the membrane with a crosslinking agent.

15. The method of claim 14, wherein the crosslinking agent is selected from glutaraldehyde, dialdehyde and formaldehyde.

16. A method of food preparation comprising contacting capsules made by the method of claim 10 with a food; and releasing the substance located in the capsule thereby forming a food product containing the substance.

17. A method as claimed in claim 16, wherein the substance is an oil or oil-phase.

18. A method as claimed in claim 16 or 17, wherein the capsules are ruptured in a cooking step, are not removed and become part of the final product.

19. A method of making a paper product comprising coating the said paper on a surface thereof with capsules made by the method of claim 10, the capsules being rupturable by pressure applied to the coated paper.

20. A method according to claim 19 wherein the capsules are ruptured and the surface of the paper is coated with the ruptured contents of the capsules.

21. A method according to claim 20, wherein the ruptured capsules form a continuous membrane on the surface of the paper product thereby forming a coated paper product.

**22.** A method according to claim 19, wherein the substance within the capsule comprises an oil in combination with a colour reactant which turns to a coloured form on contact with appropriate record material.

**Patentansprüche**
**Patentansprüche für folgende Vertragsstaaten : AT, BE, CH, DE, DK, FR, GB, GR, IT, LI, LU, NL, SE**

**1.** Unlösliche Polymerkapsel mit mindestens zwei Polymerkomponenten, wobei eine der Komponenten ein hydrophiles Kolloid ist und eine andere der Komponenten ein Abbauprodukt eines Polysaccharidderivates ist, wobei das Abbauprodukt eine Mischung von Oligomeren aufweist, von denen eine Mehrheit einen Polymerisationsgrad derart aufweist, daß die Polymere mit einer stäbchenartigen Konfiguration übereinstimmen und einen mittleren Polymerisationsgrad in dem Bereich von ungefähr 3 bis 100 aufweisen.

**2.** Kapsel nach Anspruch 1, bei der die Kapsel eine Wanddicke von weniger als ungefähr 10 $\mu$m aufweist.

**3.** Kapsel nach Anspruch 1 oder 2, bei der das Polysaccharidderivat ein Zellulosederivat, ein Stärkederivat, Carrageenan, Pullulan, Pustulan, Alginat, Laminarin, Guaran, Gummi arabicum, Inulin, Pektin, Whelan, Rhamsan, Gellan, Xanthan, Skleroglucan, Zooglan, Methylan, Chitin, Cyclodextrin oder Chitosan ist.

**4.** Kapsel nach Anspruch 3, bei der das Polysaccharidderivat einen oder mehrere Substituenten aufweist, die aus Carboxymethyl, Methyl, Hydroxypropyl, Methylethyl, Hydroxyethyl, Hydroxymethylethyl, Hydroxypropylmethyl, Sulfat, Carboxylsäure, Carboxylsäureester und Pyruvat gewählt sind.

**5.** Kapsel nach einem der Ansprüche 1 bis 4, bei der die Mischung aus Oligomeren eine Molekulargewichtsverteilung derart aufweist, daß der Polydispersie-Index der Mischung kleiner als 2 ist.

**6.** Kapsel nach einem der Ansprüche 1 bis 5, bei der die Mischung der Oligomere weniger als 25 Gewichtsprozent Mono- und Di-Saccharide enthält.

**7.** Kapsel nach einem der Ansprüche 1 bis 6, die weiter eine Substanz innerhalb der Kapsel aufweist.

**8.** Kapsel nach Anspruch 7, bei der die Substanz eine bioaktive Substanz und/oder ein Öl ist.

**9.** Kapsel nach Anspruch 8, bei der die bioaktive Substanz ein Kosmetikum, ein Lebensmittel oder ein Nahrungsmittelprodukt ist.

**10.** Kapsel nach Anspruch 8, bei der das Öl aus tierischem Öl, pflanzlichem Öl, Mineralöl und synthetischem Öl gewählt ist.

**11.** Kapsel nach einem der Ansprüche 1 bis 10, bei der das hydrophile Kolloid in der Lage ist zu geelieren und ionisierbar oder nicht-ionisierbar ist.

**12.** Kapsel nach Anspruch 11, bei der das geelierbare Kolloid Gelatine, Albumin, Gummi arabicum, ein Alginat, Casein, Agar-Agar oder ein Pektin ist.

**13.** Kapsel nach Anspruch 11 oder 12, bei der das hydrophile Kolloid eine entgegengesetzte Ladung zu der Mischung der Oligomere aufweist.

**14.** Kapsel nach einem der Ansprüche 1 bis 13, bei der die Kapselmembrane eine Dicke von 2 $\mu$m bis 10 $\mu$m aufweist.

**15.** Verfahren zum Herstellen einer Polymerkapsel, mit:
    a) Kombinieren eines hydrophilen Kolloids und eines Abbauproduktes eines Polysaccharidderivates, wobei das Abbauprodukt eine Mischung aus Oligomeren aufweist, von denen eine Mehrheit einen Polymerisationsgrad derart aufweist, daß die Polymere mit einer stäbchenartigen Konfiguration übereinstimmen und einen mittleren Polymerisationsgrad in dem Bereich von ungefähr 3 bis 300

16

aufweisen;

b) Vorsehen von Bedingungen, die für die Wechselwirkung zwischen dem hydrophilen Kolloid und der Mischung der Oligomere von Schritt a) zum Bilden von Polymerkapseln ausreichen; und

c) Entfernen der verbleibenden Polymerkomponenten von den Kapseln.

16. Verfahren nach Anspruch 15, bei dem das hydrophile Kolloid wie in Anspruch 11, 12 oder 13 definiert ist.

17. Verfahren nach Anspruch 16, bei dem das hydrophile Kolloid eine zu der Mischung von Oligomeren entgegengesetzte Ladung aufweist und der pH-Wert in Schritt b) so eingestellt wird, daß Ionen des Kolloids und der Oligomere verschiedene elektrische Ladungen aufweisen und Koazervattröpfchen oder Kapseln bilden.

18. Verfahren nach Anspruch 15, 16 oder 17, bei dem die Mischung von Oligomeren wie in einem der Ansprüche 3, 4, 5 oder 6 ist.

19. Verfahren nach einem der Ansprüche 15 bis 18, weiter mit Kombinieren der Mischung von Schritt a) mit einer Substanz, die darin einzukapseln ist, wodurch Kapseln gebildet werden, die die Substanz enthalten.

20. Verfahren nach Anspruch 19, bei dem die Substanz eine in einem der Ansprüche 8 bis 10 definierte ist.

21. Verfahren nach einem der Ansprüche 15 bis 20, weiter mit Verstärken des unlöslichen Polymerkomplexes durch Vernetzen der Membrane mit einem Vernetzungsmittel.

22. Verfahren nach Anspruch 21, bei dem das Vernetzungsmittel von Glutaraldehyd, Dialdehyd und Formaldehyd gewählt wird.

23. Verfahren zum Herstellen eines Lebensmittels mit Kontaktieren der Kapseln von Anspruch 7 mit einem Lebensmittel und Freigeben der in der Kapsel angeordneten Substanz, wodurch ein Lebensmittelprodukt gebildet wird, daß die Substanz enthält.

24. Verfahren nach Anspruch 23, bei dem die Substanz ein Öl oder eine Ölphase ist.

25. Verfahren nach Anspruch 23 oder 24, bei dem die Kapseln in einem Kochschritt zerstört werden, nicht entfernt werden und ein Teil des Endproduktes werden.

26. Papierprodukt, bei dem eine Oberfläche davon mit den Kapseln von Anspruch 7 beschichtet ist, wobei die Kapseln zerstörbar sind durch auf das beschittete Papier ausgeübten Druck.

27. Papierprodukt, bei dem eine Oberfläche davon mit den zerstörten Inhalten der Substanz und Kapseln von Anspruch 7 beschichtet ist.

28. Papierprodukt nach Anspruch 27, bei dem die zerstörten Kapseln eine kontinuierliche Membran auf der Oberfläche des Papierproduktes bilden, wodurch ein beschichtetes Papierprodukt gebildet wird.

29. Papierprodukt nach Anspruch 26, bei dem die Substanz in der Kapsel ein Öl in Kombination mit einem Farbreaktionsmittel aufweist, das sich in eine farbige Form in Kontakt mit geeignetem Aufzeichnungsmaterial umwandelt.

**Patentanprüche für folgenden Vertragsstaat : ES**

1. Verfahren zum Herstellen einer Polymerkapsel, mit:

a) Kombinieren eines hydrophilen Kolloids und eines Abbauproduktes eines Polysaccharidderivates, wobei das Abbauprodukt eine Mischung aus Oligomeren aufweist, von denen eine Mehrheit einen Polymerisationsgrad derart aufweist, daß die Polymere mit einer stäbchenartigen Konfiguration übereinstimmen und einen mittleren Polymerisationsgrad in dem Bereich von ungefähr 3 bis 300 aufweisen;

17

b) Vorsehen von Bedingungen, die für die Wechselwirkung zwischen dem hydrophilen Kolloid und der Mischung der Oligomere von Schritt a) zum Bilden von Polymerkapseln ausreichen; und
c) Entfernen der verbleibenden Polymerkomponenten von den Kapseln.

2. Verfahren nach Anspruch 1, bei dem das hydrophile Kolloid in der Lage ist, zu gelieren und ionisierbar oder nicht-ionisierbar ist.

3. Verfahren nach Anspruch 2, bei dem das gelierbare Kolloid Gelatine, Albumin, Gummi arabicum, ein Alginat, Casein, Agar-Agar oder ein Pektin ist.

4. Verfahren nach Anspruch 2 oder 3, bei dem das hydrophile Kolloid eine entgegengesetzte Ladung zu der Mischung der Oligomere aufweist.

5. Verfahren nach Anspruch 2, 3 oder 4, bei dem das hydrophile Kolloid eine zu der Mischung von Oligomeren entgegengesetzte Ladung aufweist und der pH-Wert in Schritt b) so eingestellt wird, daß Ionen des Kolloids und der Oligomere verschiedene elektrische Ladungen aufweisen und Koazervattröpfchen oder Kapseln bilden.

6. Verfahren nach einem der Ansprüche 1 bis 5, bei dem das Polysaccharidderivat ein Zellulosederivat, ein Stärkederivat, Carrageenan, Pullulan, Pustulan, Alginat, Laminarin, Guaran, Gummi arabicum, Inulin, Pektin, Whelan, Rhamsan, Gellan, Xanthan, Skleroglucan, Zooglan, Methylan, Chitin, Cyclodextrin oder Chitosan ist.

7. Verfahren nach Anspruch 6, bei dem das Polysaccharidderivat einen oder mehrere Substituenten aufweist, die aus Carboxymethyl, Methyl, Hydroxypropyl, Methylethyl, Hydroxyethyl, Hydroxymethylethyl, Hydroxypropylmethyl, Sulfat, Carboxylsäure, Carboxylsäureester und Pyruvat gewählt sind.

8. Verfahren nach einem der Ansprüche 1 bis 7, bei dem die Mischung aus Oligomeren eine Molekulargewichtsverteilung derart aufweist, daß der Polydispersie-Index der Mischung kleiner als 2 ist.

9. Verfahren nach einem der Ansprüche 1 bis 8, bei dem die Mischung der Oligomere weniger als 25 Gewichtsprozent Mono- und Di-Saccharide enthält.

10. Verfahren nach einem der Ansprüche 1 bis 9, weiter mit Kombinieren der Mischung von Schritt a) mit einer Substanz, die darin einzukapseln ist, wodurch Kapseln gebildet werden, die die Substanz enthalten.

11. Verfahren nach Anspruch 10, bei dem die Substanz eine bioaktive Substanz und/oder ein Öl ist.

12. Verfahren nach Anspruch 11, bei dem die bioaktive Substanz ein Kosmetikum, ein Lebensmittel oder ein Nahrungsmittelprodukt ist.

13. Verfahren nach Anspruch 11, bei dem das Öl aus tierischem Öl, pflanzlichem Öl, Mineralöl und synthetischem Öl gewählt ist.

14. Verfahren nach einem der Ansprüche 1 bis 13, weiter mit Verstärken des unlöslichen Polymerkomplexes durch Vernetzen der Membrane mit einem Vernetzungsmittel.

15. Verfahren nach Anspruch 14, bei dem das Vernetzungsmittel von Glutaraldehyd, Dialdehyd und Formaldehyd gewählt wird.

16. Verfahren zum Herstellen eines Lebensmittels mit Kontaktieren der Kapseln von Anspruch 10 mit einem Lebensmittel und Freigeben der in der Kapsel angeordneten Substanz, wodurch ein Lebensmittelprodukt gebildet wird, das die Substanz enthält.

17. Verfahren nach Anspruch 16, bei dem die Substanz ein Öl oder eine Ölphase ist.

**18.** Verfahren nach Anspruch 16 oder 17, bei dem die Kapseln in einem Kochschritt zerstört werden, nicht entfernt werden und ein Teil des Endproduktes werden.

**19.** Verfahren zum Herstellen eines Papierproduktes, bei dem eine Oberfläche davon mit den Kapseln von Anspruch 10 beschichtet wird, wobei die Kapseln zerstörbar sind durch auf das beschichtete Papier ausgeübten Druck.

**20.** Verfahren nach Anspruch 19, bei dem Kapseln zerstört werden und die Oberfläche des Papieres mit den zerstörten Inhalten der Kapseln beschichtet wird.

**21.** Verfahren nach Anspruch 20, bei dem die zerstörten Kapseln eine kontinuierliche Membran auf der Oberfläche des Papierproduktes bilden, wodurch ein beschichtetes Papierprodukt gebildet wird.

**22.** Verfahren nach Anspruch 19, bei dem die Substanz in der Kapsel ein Öl in Kombination mit einem Farbreaktionsmittel aufweist, das sich in eine farbige Form in Kontakt mit geeignetem Aufzeichnungsmaterial umwandelt.

**Revendications**

**Revendications pour les Etats contractants suivants : AT, BE, CH, DE, DK, FR, GB, GR, IT, LI, LU, NL, SE**

**1.** Capsule insoluble à base de polymères, ayant au moins deux composants polymères, dans laquelle l'un desdits composants est un colloïde hydrophile et un autre desdits composants est un produit de dégradation d'un dérivé de polysaccharide, ledit produit de dégradation comprenant un mélange d'oligomères dont la majorité a un degré de polymérisation tel que les oligomères présentent une configuration de type baguette et un degré moyen de polymérisation situé dans l'intervalle compris entre 3 et 100.

**2.** Capsule selon la revendication 1, dans laquelle la capsule présente une épaisseur de paroi inférieure à environ 10 microns.

**3.** Capsule selon la revendication 1 ou 2, dans laquelle le dérivé de polysaccharide est un dérivé de cellulose, un dérivé d'amidon, le carraghénane, le pullulane, le pustulane, un alginate, la laminarine, la gomme de guar, la gomme arabique, l'inuline, la pectine, le whelane, le rhamsane, le gellane, le xanthane, le scléroglucane, le zooglane, le méthylane, la chitine, la cyclodextrine ou le chitosane.

**4.** Capsule selon la revendication 3, dans laquelle le dérivé de polysaccharide comprend un ou plusieurs substituants choisis parmi les groupes carboxyméthyle, méthyle, hydroxypropyle, méthyléthyle, hydroxyéthyle, hydroxyméthyléthyle, hydroxypropylméthyle, sulfate, acide carboxylique, ester d'acide carboxylique et pyruvate.

**5.** Capsule selon l'une quelconque des revendications 1 à 4, dans laquelle le mélange d'oligomères présente une distribution de la masse moléculaire telle que l'indice de polydispersion du mélange est inférieur à 2.

**6.** Capsule selon l'une quelconque des revendications 1 à 5, dans laquelle le mélange d'oligomères contient moins de 25 % en poids de mono- et disaccharides.

**7.** Capsule selon l'une quelconque des revendications 1 à 6, comprenant en outre une substance à l'intérieur de la capsule.

**8.** Capsule selon la revendication 7, dans laquelle la substance est une substance bioactive et/ou une huile.

**9.** Capsule selon la revendication 8, dans laquelle la substance bioactive est un produit cosmétique, alimentaire ou d'alimentation animale.

**10.** Capsule selon la revendication 8, dans laquelle l'huile est choisie parmi les huiles animales, végétales, minérales et de synthèse.

**11.** Capsule selon l'une quelconque des revendications 1 à 10, dans laquelle le colloïde hydrophile est capable de se gélifier, et il est ionisable ou non-ionisable.

**12.** Capsule selon la revendication 11, dans laquelle le colloïde gélifiable est la gélatine, l'albumine, la gomme arabique, un alginate, la caséine, l'agar-agar ou une pectine.

**13.** Capsule selon la revendication 11 ou 12, dans laquelle le colloïde hydrophile présente une charge opposée à celle dudit mélange d'oligomères.

**14.** Capsule selon l'une quelconque des revendications 1 à 13, dans laquelle la membrane capsulaire présente une épaisseur de 2 $\mu$ à 10 $\mu$.

**15.** Procédé de préparation d'une capsule à base de polymères, comprenant les étapes consistant à :
a) combiner un colloïde hydrophile et un produit de dégradation d'un dérivé de polysaccharide, ledit produit de dégradation comprenant un mélange d'oligomères dont la majorité a un degré de polymérisation tel que les oligomères présentent une configuration de type baguette, et un degré moyen de polymérisation situé dans l'intervalle d'environ 3 à 100 ;
b) fournir des conditions suffisantes pour l'interaction entre le colloïde hydrophile et le mélange d'oligomères de l'étape a) pour former des capsules à base de polymères ; et
c) éliminer des capsules les composants polymères résiduels.

**16.** Procédé selon la revendication 15, dans lequel le colloïde hydrophile est tel que défini dans la revendication 11, 12 ou 13.

**17.** Procédé selon la revendication 16, dans lequel le colloïde hydrophile présente une charge opposée à celle du mélange d'oligomères et le pH est ajusté dans l'étape b) de façon que les ions dudit colloïde et desdits oligomères présentent des charges électriques différentes et forment des gouttelettes ou capsules coacervées.

**18.** Procédé selon la revendication 15, 16 ou 17, dans lequel ledit mélange d'oligomères est tel que défini dans l'une quelconque des revendications 3, 4, 5 ou 6.

**19.** Procédé selon l'une quelconque des revendications 15 à 18, comprenant en outre l'étape consistant à combiner le mélange de l'étape a) avec une substance à encapsuler, formant ainsi des capsules contenant la substance.

**20.** Procédé selon la revendication 19, dans lequel la substance est telle que définie dans l'une quelconque des revendications 8 à 10.

**21.** Procédé selon l'une quelconque des revendications 15 à 20, comprenant en outre l'étape de renforcement du complexe polymère insoluble, par réticulation de la membrane à l'aide d'un agent de réticulation.

**22.** Procédé selon la revendication 21, dans lequel l'agent de réticulation est choisi parmi le glutaraldéhyde, le dialdéhyde et le formaldéhyde.

**23.** Procédé pour la préparation d'un produit alimentaire, comprenant les étapes consistant à : mettre en contact des capsules selon la revendication 7 avec un produit alimentaire ; et libérer la substance se trouvant dans les capsules, formant ainsi un produit alimentaire contenant la substance.

**24.** Procédé selon la revendication 23, dans lequel la substance est une huile ou une phase huileuse.

**25.** Procédé selon la revendication 23 ou 24, dans lequel les capsules sont rompues dans une étape culinaire, ne sont pas élliminées et entrent dans la composition du produit final.

**26.** Produit papetier dont la surface est revêtue de capsules selon la revendication 7, les capsules pouvant être rompues par une pression appliquée sur le papier couché.

**27.** Produit papetier dont la surface est revêtue du contenu de la substance et des capsules éclatées selon la revendication 7.

**28.** Produit papetier selon la revendication 27, dans lequel les capsules éclatées forment une membrane continue à la surface du produit papetier, constituant ainsi un produit papetier couché.

**29.** Produit papetier selon la revendication 26, dans lequel la substance à l'intérieur de la capsule comprend une huile en combinaison avec un réactif coloré, qui prend sa forme colorée au contact d'un matériau d'enregistrement approprié.

**Revendications pour l'Etat contractant suivant : ES**

**1.** Procédé de préparation d'une capsule à base de polymères, comprenant les étapes consistant à :
a) combiner un colloïde hydrophile et un produit de dégradation d'un dérivé de polysaccharide, ledit produit de dégradation comprenant un mélange d'oligomères dont la majorité a un degré de polymérisation tel que les oligomères présentent une configuration de type baguette, et un degré moyen de polymérisation situé dans l'intervalle d'environ 3 à 100 ;
b) fournir des conditions suffisantes pour l'interaction entre le colloïde hydrophile et le mélange d'oligomères de l'étape a) pour former des capsules à base de polymères ; et
c) éliminer des capsules les composants polymères résiduels.

**2.** Procédé selon la revendication 1, dans lequel le colloïde hydrophile est capable de se gélifier, et est ionisable ou non-ionisable.

**3.** Procédé selon la revendication 2, dans lequel le colloïde gélifiable est la gélatine, l'albumine, la gomme arabique, un alginate, la caséine, l'agar-agar ou une pectine.

**4.** Procédé selon la revendication 2 ou 3, dans lequel le colloïde hydrophile présente une charge opposée à celle dudit mélange d'oligomères.

**5.** Procédé selon l'une quelconque des revendications 2, 3 ou 4, dans lequel le colloïde hydrophile présente une charge opposée à celle du mélange d'oligomères et le pH est ajusté dans l'étape b) de façon que les ions dudit colloïde et desdits oligomères présentent des charges électriques différentes et forment des gouttelettes ou capsules coacervées.

**6.** Procédé selon l'une quelconque des revendications 1 à 5, dans lequel le dérivé de polysaccharide est un dérivé de la cellulose, un dérivé d'amidon, le carraghénane, le pullulane, le pustulane, un alginate, la laminarine, la gomme de guar, la gomme arabique, l'inuline, la pectine, le whelane, le rhamsane, le gellane, le xanthane, le scléroglucane, le zooglane, le méthylane, la chitine, la cyclodextrine ou le chitosane.

**7.** Procédé selon la revendication 6, dans lequel le dérivé de polysaccharide comprend un ou plusieurs substituants choisis parmi les groupes carboxyméthyle, méthyle, hydroxypropyle, méthyléthyle, hydroxyéthyle, hydroxyméthyléthyle, hydroxypropylméthyle, sulfate, acide carboxylique, ester d'acide carboxylique et pyruvate.

**8.** Procédé selon l'une quelconque des revendications 1 à 7, dans lequel le mélange d'oligomères présente une distribution de la masse moléculaire telle que l'indice de polydispersion du mélange est inférieur à 2.

**9.** Procédé selon l'une quelconque des revendications 1 à 8, dans lequel le mélange d'oligomères contient moins de 25 % en poids de mono- et disaccharides.

**10.** Procédé selon l'une quelconque des revendications 1 à 9, comprenant en outre l'étape consistant à combiner le mélange de l'étape a) avec une substance à encapsuler, formant ainsi des capsules

21

contenant la substance.

11. Procédé selon la revendication 10, dans lequel la substance est une substance bioactive et/ou une huile.

12. Procédé selon la revendication 11, dans lequel la substance bioactive est un produit cosmétique, alimentaire ou d'alimentation animale.

13. Procédé selon la revendication 11, dans lequel l'huile est choisie parmi les huiles animales, végétales, minérales et de synthèse.

14. Procédé selon l'une quelconque des revendications 1 à 13, comprenant en outre l'étape de renforcement du complexe polymère insoluble, par réticulation de la membrane à l'aide d'un agent de réticulation.

15. Procédé selon la revendication 14, dans lequel l'agent de réticulation est choisi parmi le glutaraldéhyde, le dialdéhyde et le formaldéhyde.

16. Procédé pour la préparation d'un produit alimentaire, comprenant les étapes consistant à : mettre en contact des capsules obtenues par le procédé selon la revendication 10 avec un produit alimentaire ; et libérer la substance se trouvant dans les capsules, formant ainsi un produit alimentaire contenant la substance.

17. Procédé selon la revendication 16, dans lequel la substance est une huile ou une substance huileuse.

18. Procédé selon la revendication 16 ou 17, dans lequel les capsules sont rompues dans une étape culinaire, ne sont pas éliminées et entrent dans la composition du produit final.

19. Procédé de préparation d'un produit papetier, consistant à revêtir la surface dudit papier de capsules obtenues par le procédé selon la revendication 10, les capsules pouvant être rompues par une pression appliquée sur le papier couché.

20. Procédé selon la revendication 19, dans lequel les capsules sont rompues et la surface du papier est revêtue avec le contenu des capsules éclatées.

21. Procédé selon la revendication 20, dans lequel les capsules éclatées forment une membrane continue à la surface du produit papetier, constituant ainsi un produit papetier couché.

22. Procédé selon la revendication 19, dans lequel la substance à l'intérieur des capsules comprend une huile en combinaison avec un réactif coloré qui prend sa forme colorée au contact d'un matériau d'enregistrement approprié.

## Fig.1

```
   0.1 – 5%                          0.1 – 30%
  HYDROPHILIC                      POLYSACCHARIDE
    POLYMER                          DERIVATIVES
        └──────────────┬──────────────────┘
                       │
            ADJUSTMENT OF SOLUTION
               TO PRESERVE
     INTERMOLECULAR INTERACTION POTENTIAL
                       │
             ADDITION OF SUBSTANCE
                       │
             FORMATION OF EMULSION
                       │
            ADJUSTMENT OF SOLUTION
               TO ENHANCE
        INTERMOLECULAR INTERACTIONS
                       │
          FURTHER SOLIDIFICATION OF
             CAPSULE MEMBRANE
                       │
        WASHING WITH DEIONIZED WATER
                       │
                 CROSSLINKING
                       │
        WASHING WITH DEIONIZED WATER
                       │
                 DECANTATION
          ┌────────────┴────────────┐
       SLURRY                      DRYING
```

EP 0 470 872 B1

## Fig. 2

24

# Fig. 3

Fig. 4

## Fig. 5